# EUROPÄISCHE PATENTANMELDUNG

(11) **EP 0 800 825 A1**
(43) Veröffentlichungstag der Anmeldung: **15.10.1997**
(21) Anmeldenummer: 97103484.8
(22) Anmeldetag: 04.03.1997
(51) Int. Cl.: A61K 31/07, A61K 31/355, A61K 31/375, A61K 9/08

(54) **Stabile wässrige Solubilisate von Carotinoiden und Vitamine**

(30) Priorität: 11.03.1996 DE 19609477
(71) Anmelder: BASF AKTIENGESELLSCHAFT, 67056 Ludwigshafen (DE)
(72) Erfinder: Kolter, Karl, Dr., 67117 Limburgerhof (DE); Runge, Frank, Dr., 67133 Maxdorf (DE)

(57) **Zusammenfassung**

Stabile, zur parenteralen Verabreichung geeignete wäßrige Solubilisate von Carotinoiden und Vitaminen oder Vitaminderivaten, in denen das Carotinoid und die nicht wasserlöslichen Vitamine mit Hilfe eines nichtionogenen Emulgators micellar gelöst sind, wobei die Micellen kleiner 100 nm sind, wobei die Solubilisate, in Mengen bezogen auf das Solubilisat, zumindest ein Carotinoid in einer Konzentration von 0,1 bis 10 Gew.-%, die nicht wasserlöslichen, lipophilen Vitamine oder Vitaminderivate in einer Konzentration von 0,1 bis 20 Gew.-% und die nichtionogenen Emulgatoren in einer Konzentration von 1 bis 40 Gew.-% enthalten, mit der Maßgabe, daß der Gehalt der lipophilen Vitamine mindestens so groß wie der des Carotinoids ist.

## Beschreibung

Die Erfindung betrifft stabile, zur parenteralen Verabreichung geeignete wäßrige Solubilisate von Carotinoiden und Vitaminen, die mit Hilfe von nichtionogenen Emulgatoren hergestellt sind.

Carotinoide, Vitamine und Spurenelemente besitzen ernährungsphysiologisch große Bedeutung. Eine nicht ausreichende Zufuhr führt im menschlichen und tierischen Organismus zu Mangelerscheinungen. Bei einigen Erkrankungen können die Patienten oral keine Nahrung oder Medikamente aufnehmen, sondern müssen parenteral ernährt werden. In diesen Fällen müssen auch die o.g. Stoffe auf diese Weise zugeführt werden. Ferner gibt es auch Fälle, in denen Patienten auch bei oraler Ernährung stark an Carotinoiden, Vitaminen und Spurenelementen depletiert sind, so daß eine schnelle Wiederherstellung der physiologischen Konzentrationen erwünscht ist. Auch in solchen Fällen ist eine parenterale Gabe angezeigt. Darüber hinaus werden einigen Carotinoiden, Vitaminen und Spurenelementen bei Applikation von über die physiologischen Werte hinausgehenden Mengen präventive Wirkungen im Hinblick auf verschiedene Erkrankungen wie z.B. Atheriosklerose, Herzinfarkt, Stroke, Maculadegeneration, Grauer Star, Morbus Parkinson, Krebs zugeschrieben. Dabei wird davon ausgegangen, daß die antioxidativen, radikalfangenden Eigenschaften dieser Stoffe für diese Wirkungen verantwortlich sind, da bekannt ist, daß oxidative Agenzien und Radikale zu Zellveränderungen führen. Ferner sollen diese für den Menschen essentiellen Verbindungen das Immunsystem stärken. So kam es unter Gabe von Vitamin E zu einem Anstieg der T-Lymphozyten, insbesondere der T-Helferzellen.

Hierbei sind β-Carotin und weitere Carotinoide, Tocopherol bzw. Tocopherolderivate, Ascorbinsäure bzw. Ascorbinsäurederivate und Selenverbindungen von besonderer Bedeutung. Eine Kombination dieser Stoffe oder eines Teils dieser Stoffe wird üblicherweise als Antioxidanskombination bezeichnet.

Bisher ist keine zur parenteralen Applikation geeignete Formulierung verfügbar, die es erlaubt, physiologische oder über physiologische Werte hinausgehende Mengen an oben genannten Carotinoiden und Vitaminen zu verabreichen.

Aus den Europäischen Patentschriften EP 0 055 817 und der Offenlegungsschrift DE 40 31 094 A1 sind β-Carotinsolubilisate bekannt, die als Solubilisator ein nichtionogenes Tensid mit einem HLB-Wert von 12 bis 16 enthalten.

In der PCT-Anmeldung WO 94 06 310 A1 wird die Herstellung eines Solubilisates aus β-Carotin und einem Emulgator mit einem HLB-Wert von 10 bis 18 beschrieben, das zu Färbezwecken in Lebensmitteln eingesetzt werden soll. Zur Stabilisierung des β-Carotins können der Zubereitung wie üblich geringe Mengen an Antioxidantien Zugesetzt werden.

Es bestand nun die Aufgabe, Solubilisate vorzuschlagen, die sowohl β-Carotin als auch Vitamine enthalten und die stabil gegen Ausfällungen und zur parenteralen Verabreichung geeignet sind. Daher war zu erwarten, daß die Inkorporation von weiteren lipophilen Bestandteilen in Mengen, die mindestens ebenso groß wie die β-Carotinmenge oder größer sind, die Micellstruktur des solubilisierten β-Carotins derart stört, daß Ausfällungen des an sich wasserunlöslichen β-Carotins auftreten. Eine Störung war gleichermaßen von größeren Mengen wasserlöslicher Vitamine, die in der Regel in Salzform vorliegen, zu erwarten.

Es wurde nun überraschenderweise gefunden, daß zur parenteralen Verabreichung geeignete wäßrige Solubilisate von Carotinoiden und Vitaminen oder Vitaminderivaten, in denen das Carotinoid und die nicht wasserlöslichen Vitamine mit Hilfe eines nichtionogenen Emulgators micellar gelöst sind, wobei die Micellen kleiner 100 nm sind und über längere Zeit physikalisch und chemisch vollkommen stabil sind, wobei die Solubilisate, in Mengen bezogen auf das Solubilisat, zumindest ein Carotinoid in einer Konzentraton von 0,1 bis 10 Gew.-%, die nicht wasserlöslichen, lipophilen Vitamine oder Vitaminderivate in einer Konzentration von 0,1 bis 20 Gew.-% und die nicht-ionogenen Emulgatoren in einer Konzentration von 1 bis 40 Gew.-% enthalten, mit der Maßgabe, daß der Gehalt der lipophilen Vitamine mindestens so groß wie der des Carotinoids ist.

Es wurde weiter gefunden, daß aufgrund von unerwarteten Wechselwirkungen zwischen den Carotinoiden, den lipophilen Vitaminen insbesondere dem Tocopherol- bzw. Tocopherolester und dem nichtionischen Tensid die Menge an nichtionischem Tensid in der Mischung geringer gehalten werden kann als der Summe in Solubilisaten mit den einzelnen Wirkstoffen entspricht. Die Zubereitung wird dadurch für den Menschen lokal und systemisch verträglich. Hinzu kommt, daß überraschenderweise die Kombination von Carotinoiden mit lipophilen Vitaminen bei der Herstellungsweise ermöglicht, die Temperaturbelastung der Carotinoide und damit eine mögliche Zersetzung zu reduzieren. Die Solubilisierung verläuft schneller als ohne Zusatz des lipophilen Vitamins.

Dieser überraschende Befund der hervorragenden Stabilisierung wird durch die Ausbildung von Mischmizellen vermutet, in denen Wechselwirkungen auf molekularer Ebene möglich sind.

Die Solubilisate haben in der Regel mittlere Teilchengrößen der Micellen, von 5 bis 100 nm. Diese Teilchengrößen sind damit weit niedriger als die Mindestanforderung von 1 µm für Injektionsemulsionen.

Als nichtionogene Emulgatoren kommen die an sich bekannten physiologisch verträglichen Verbindungen, insbesondere solche mit einem HLB-Wert von 10 bis 20 in Betracht. Im einzelnen sind Polyoxyethylenglyceroltriricinoleat mit 20 bis 60 Oxyethyleneinheiten, Polyoxyethylen-12-hydroxystearat mit 10 bis 40 Oxyethyleneinheiten, Polyoxyethylensorbitanfettsäureester mit 10 bis 40 Oxyethyleneinheiten und Polyoxyethylen-polyoxypropylen-Block-copolymere mit der Formel 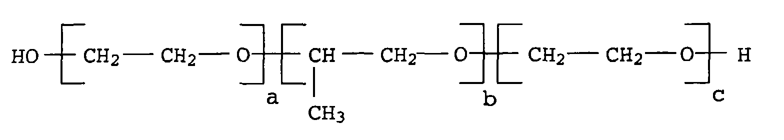 in der a und c 10 bis 130, vorzugsweise ca. 80, und b 15 bis 70, vorzugsweise ca. 30 Einheiten bedeutet, zu nennen.

Als Carotinoide kommen vor allem β-Carotin, ferner Lycopin, Astaxanthin, Canthaxanthin, Citranaxanthin, Zeaxanthin, Apocarotinal und/oder Apocarotinsäureester, als lipophile Vitamine Tocopherol, Tocopherolacetat, Tocopherolsuccinat, Retinal, Retinol, Retinolester, Retinsäure, Cholecalciferol und/oder Ergocalciferol in Betracht.

Die Carotinoide sind bevorzugt in Mengen, bezogen auf das Solubilisat, von 0,4 bis 6 Gew.-%, die lipophilen Vitamine in Mengen von 0,4 bis 10 Gew.-% und die Emulgatoren in Mengen von 5 bis 25 Gew.-% enthalten.

Für die Anwendung in Form von Ampullen, Fertigspritzen, Infusionslösungen, Tropflösungen oder Säften können die Solubilisate selbstverständlich noch mit einem physiologisch verträglichen Vehikel weiter verdünnt werden.

In der wäßrigen Phase können ferner hydrophile Vitamine wie Vitamin C oder die Vitamine der B-Reihe sowie gegebenenfalls Mineralstoffe und Spurenelemente z.B. Selenverbindungen enthalten sein. Schließlich können weitere pharmazeutisch wirksame Stoffe, wie N-Acetylcystein in den Solubilisaten vorliegen.

Eine bevorzugte Antioxidans-Kombination enthält β-Carotin, Tocopherol oder Tocopherolester, Ascorbinsäure, sowie gegebenenfalls Selenverbindungen und N-Acetylcystein.

Die Herstellung der erfindungsgemäßen Zubereitungen erfolgt in der Regel so, daß das Carotinoid mit den lipophilen Vitaminen und dem Emulgator kurzzeitig auf Temperaturen über 1200°C erhitzt wird, dadurch in Lösung geht und sofort anschließend mit einer Losung der hydrophilen Einsatzstoffe in Wasser oder einer Pufferlösung gemischt und dadurch abgekühlt wird. Alternativ kann auch mit Wasser oder einer Pufferlösung gemischt und die hydrophilen Vitamine später zugesetzt werden. Eine weitere Möglichkeit besteht darin, das Carotinoid mit dem Emulgator kurzfristig auf Temperaturen über 1200°C zu erhitzen und diese Lösung mit einem separat, in bekannter Weise hergestellten Solubilisat der lipophilen Vitamine in Wasser oder einem Puffer zu mischen.

Die bevorzugte Herstellung erfolgt diskontinuierlich nach dem Verfahren der EP 0 055 817 und insbesondere kontinuierlich nach dem Verfahren der EP 0 479 066. Deshalb wird hiermit auf diese beiden Patentschriften und die dort angegebenen Bedingungen ausdrücklich Bezug genommen.

Nach Abkühlung wird das Solubilisat - sofern es für parenterale Zwecke verwendet werden soll - sterilfiltriert z.B. durch einen 0,22 µm-Filter und in Ampullen, Fertigspritzen, Vials oder Infusionsflaschen abgefüllt. Bei oraler Verabreichung kann die Sterilfiltration entfallen, und das Solubilisat wird in Tropfflaschen oder Saftflaschen konfektioniert. In diesem Fall ist es vorteilhaft zur Verbesserung des Geschmackes während der Herstellung des Solubilisats oder anschließend Süßungsmittel und Aromastoffe zuzusetzen.

### Beispiele

### Beispiel 1

In einen mit Stickstoff begasten Kolben wurden 23,0 g Polyoxyethylen-(12-hydroxystearat mit 15 Oxyethylen-Einheiten (Solutol® HS 15), 5,0 g Tocopherolacetat und 0,5 g Butylhydroxitoluol eingetragen und auf 180°C erhitzt. Unter Rühren wurden anschließend 6,0 g β-Carotin aufgelöst, die Heizung entfernt und die heiße Mischung mit einer ca. 20°C warmen Lösung aus 4,9 g Natriumascorbat und 0,1 g Ascorbinsäure in 60,5 g Wasser für Injektionszwecke unter turbulenter Vermischung versetzt. Dabei entstand ein klares, tiefrotes, dünnflüssiges Solubilisat, das durch einen Filter mit einer Porenweite von 0,45 µm filtriert und in Vials mit Gummistopfen abgefüllt wurde.
- Gehalt β-Carotin:: 5,8 %
- Gehalt Tocopherolacetat:: 5,1 %
- Gehalt Ascorbinsäure:: 4,2 %
- Mizellgröße:: 32 nm
- pH-Wert:: 5,8

Die Zubereitung war auch nach 6-monatiger Lagerung bei Raumtemperatur und 30°C absolut klar und unverändert in ihren Gehaltswerten.

### Beispiel 2

In einem mit Stickstoff begasten Kolben wurden 23,0 g Solutol® HS 15 und 5,0 g Tocopherol auf 180°C erhitzt. Unter Rühren wurden anschließend 4,0 g β-Carotin aufgelöst, die Heizung entfernt und die heiße Mischung mit einer 20°C warmen Lösung aus 4,9 g Natriumascorbat und 0,1 g Ascorbinsäure in 63,0 g Wasser für Injektionszwecke versetzt. Dabei entsteht ein klares, tiefrotes, dünnflüssiges Solubilisat, das durch einen Filter mit einer Porenweite von 0,45 µm filtriert und in Vials mit Gummistopfen abgefüllt wurde.

Die Zubereitung war auch nach 6-monatiger Lagerung bei Raumtemperatur und 30°C absolut klar und unverändert in ihren Gehaltswerten.
- Gehalt β-Carotin:: 3,9 %
- Gehalt Tocopherolacetat:: 5,2 %
- Gehalt Ascorbinsäure:: 4,3 %
- Mizellgröße:: 27 nm
- pH-Wert:: 6,0

### Beispiel 3

In einem mit Stickstoff begasten Kolben wurden 23,0 g Solutol® HS 15 und 10,0 g Tocopherolacetat und 0,5 g Tocopherol auf 180°C erhitzt. Unter Rühren wurden anschließend 6,0 g β-Carotin aufgelöst, die Heizung entfernt und die heiße Mischung mit einer 20°C warmen Lösung aus 5,5 g Natriumascorbat und 0,1 g Ascorbinsäure in 54,0 g Wasser für Injektionszwecke versetzt. Das klare, tiefrote Solubilisat wurde nach Abkühlung auf Raumtemperatur durch einen Filter mit einer Porenweite von 0,45 µm filtriert und in Ampullen abgefüllt.
- Gehalt β-Carotin:: 5,7 %
- Gehalt Tocopherolacetat:: 10,4 %
- Gehalt Ascorbinsäure:: 5,1 %
- Mizellgröße:: 42 nm
- pH-Wert:: 6,0

### Beispiel 4

### Kontinuierliche Herstellung

In einer beheizten Vorlage wurden bei einer Temperatur von 70°C eine Suspension von 40 g β-Carotin in 250 g Solutol® HS 15, 50 g Tocopherolacetat und 10 g Butylhydroxytoluol vorgelegt. Diese Suspension wurde mittels einer Hochdruckpumpe bei einem Durchsatz von 2 l/h einer in ein Ölbad eingetauchten Heizschlange zugeführt. Bei einem inneren Durchmesser von 2 mm und einer Länge von 6 m wurde bei Wärmeträgeröl-Temperaturen von 170°C eine Verweilzeit von 34 sec eingestellt. Diese Zeit reicht aus, um das β-Carotin zu lösen. Nach der genannten Verweilzeit in der Heizschlange trat die β-Carotin-Lösung in eine T-förmige Mischkammer ein, in der unter einem Mischungswinkel von 180°C über eine Hochdruckpumpe eine wäßrige Lösung mit 6,8 % Natriumascorbat, 0,2 % Ascorbinsäure und 0,01 % Thimerosal bei einem Durchsatz von 4,7 l/h turbulent zugemischt wurden. Bei einem Druck von 25 bar wurde das Produkt über ein Druckbegrenzer-Ventil ausgetragen. Es wurde eine dunkelrot gefärbte, mizellare Antioxidans-Lösung erhalten.

Nach Filtration durch einen 0,45 µm-Filter wurde das Solubilisat unter Stickstoffbegasung in Vials mit Gummistopfen abgefüllt.
- Gehalt β-Carotin:: 3,3 %
- Gehalt Tocopherolacetat:: 4,3 %
- Gehalt Ascorbinsäure:: 4,3 %
- Mizellgröße:: 28 nm
- pH-Wert:: 5,9

### Beispiel 5

In einen mit Stickstoff begasten Kolben wurden 13,0 g Solutol® HS 15 und 0,5 g Tocopherol eingetragen und auf 180°C erhitzt. Unter Rühren wurden anschließend 4,0 g β-Carotin aufgelöst, die Heizung entfernt und die heiße Mischung mit 82,5 g eines separat hergestellten Tocopherolacetat-Ascorbinsäure-Solubilisates versetzt.

Zur Herstellung des Tocopherolacetat-Ascorbinsäuresolubilisates wurden 5,0 g Tocopherolacetat mit 10,0 g Solutol® HS 15 gemischt und auf 65°C erwärmt. Unter innigem Rühren wurde in dieses Gemisch langsam eine Lösung von 4,9 g Natriumascorbat und 0,1 g Ascorbinsäure in 62,5 g demineralisiertem Wasser eingearbeitet.

Das klare, tiefrote Antioxidanssolubilisat wurde nach Abkühlung auf Raumtemperatur durch einen 0,45 µm-Filter filtriert und in Vials mit Gummistopfen abgefüllt.
- Gehalt β-Carotin:: 3,9 %
- Gehalt Tocopherolacetat:: 5,2 %
- Gehalt Ascorbinsäure:: 4,3 %
- Mizellgröße:: 29 nm
- pH-Wert:: 6,1

### Beispiel 6

In einer auf 80°C temperierten Vorlage wurden 20,0 g Tocopherolacetat und 0,5 g Tocopherol mit 200 g Solutol® HS 15 gemischt. Anschließend wurden 10,0 g β-Carotin gleichmäßig unter Stickstoffbegasung suspendiert. Diese Suspension wurde mittels einer Hochdruckpumpe bei einem Durchsatz von 2 l/h einer in ein Ölbad eingetauchten Heizschlange zugeführt. Die Heizbadtemperatur beträgt 170°C und die Verweilzeit in der Heizschlange ca. 34 sec. Nach Verlassen der Heizschlange wurde die klare, rote Lösung in einem mit Stickstoff begasten Kolben aufgefangen. In diese Lösung wurde anschließend eine separat hergestellte Lösung von 100,0 g Natriumascorbat, 40,0 g Nicotinsäureamid, 15,0 g Pydridoxin HCl, 10,0 g Riboflavin-5-phosphat-Natrium x 2H₂O, 10,0 g Thiamin-HCl und 25,0 g Dexpanthenol in einer Mischung aus 406,0 g 0,1-molarer Natronlauge und 1 164,0 g Wasser für Injektionszwecke langsam eingerührt, die Zubereitung auf Raumtemperatur abgekühlt und durch einen 0,45 µm-Filter filtriert.
- Gehalt β-Carotin:: 0,48 %
- Gehalt Tocopherolacetat:: 1,05 %
- Gehalt Natriumascorbat:: 4,9 %
- Gehalt Riboflavin-5-phosphat-Natrium x 2H₂O:: 0,50 %
- Gehalt Thiamin-HCl:: 0,46 %
- Gehalt Nicotinsäureamid:: 2,10 %
- Gehalt Pyridoxin-HCl:: 0,74 %
- Mizellgröße:: 19 nm
- pH-Wert:: 5,5

### Beispiel 7 (Vergleichsversuch)

Mit der kontinuierlichen Herstellungsmethode wie in Beispiel 4 beschrieben wurden folgende Versuche durchgeführt:

| | | |
|---|---|---|
| Rezeptur A: | β-Carotin | 6 % |
| | Solutol® HS 15 | 23 % |
| | Wasser für Injektionszwecke ad | 100 % |
| Rezeptur B: | β-Carotin | 6 % |
| | Tocopherol | 1,2 % |
| | Solutol® HS 15 | 23 % |
| | Wasser für Injektionszwecke ad | 100 % |
| Rezeptur C: | β-Carotin | 6 % |
| | Tocopherolacetat | 10 % |
| | Solutol® HS 15 | 23 % |
| | Wasser für Injektionszwecke ad | 100 % |

Die Ölbadtemperatur betrug jeweils 170°C.

### Ergebnisse:

- Rezeptur A:: trübes, nicht ausreichend stabiles Solubilisat
- Rezeptur B:: trübes, nicht ausreichend stabiles Solubilisat
- Rezeptur C:: klares, stabiles Solubilisat

Erst der Zusatz größerer Mengen öllöslicher Vitamine ermöglicht die Herstellung eines stabilen Solubilisates vor allem bei relativ hohen β-Carotin Konzentrationen.

### Beispiel 8

In einem mit Stickstoff begasten Kolben wurden 23,0 g Solutol® HS 15 und 5 g Tocopherol auf 180°C erhitzt. Unter Rühren wurden anschließend 4,0 g β-Carotin aufgelöst, die Heizung entfernt und die heiße Mischung mit einer 20°C warmen Lösung aus 5,5 g Natriumascorbat und 0,1 g Ascorbinsäure in 62,4 g Wasser für Injektionszwecke versetzt. Das klare, tiefrote Solubilisat wurde nach Abkühlung auf Raumtemperatur durch einen Filter mit einer Porenweite von 0,45 µm filtriert und in Vials abgefüllt.
- Gehalt β-Carotin:: 3,8 %
- Gehalt Tocopherol:: 5,0 %
- Gehalt Ascorbinsäure:: 5,2 %
- Mizellgröße:: 27 nm
- pH-Wert:: 6,0

### Beispiel 9

Analog zu der in Beispiel 4 beschriebenen kontinuierlichen Herstellungsmethode wurde eine Suspension von 16 g Astaxanthin in 400 g Solutol® HS 15 und 32 g Tocopherolacetat bei einer Temperatur von 60°C vorgelegt. Bei einem Durchsatz von 2,2 l/h wurde die Suspension durch eine Heizschlange mit einem Innendurchmesser von 2 mm und einer Länge von 12 m geleitet, die in ein Ölbad mit einer Temperatur von ca. 210°C eingetaucht war. Die so eingestellte Verweilzeit von 62 Sekunden reichte aus, um das Astaxanthin in dem Emulgator zu lösen. In der Mischkammer wurde danach eine wäßrige Lösung von 1,5 g/l Ascorbinsäure und 37 g/l Natriumascorbat bei einem Durchsatz von 5,4 l/h turbulent zugemischt. Das Produkt wurde bei einem Druck von 30 bar über ein Druckbegrenzer-Ventil ausgetragen. Es wurde eine dunkelrot gefärbte, mizellare Astaxanthin-Lösung erhalten, die durch einen 0,22 µm-Filter filtriert wurde.
- Gehalt Astaxanthin:: 0,8 %
- Gehalt Tocopherolacetat:: 2,0 %
- Gehalt: Natriumascorbat+Ascorbinsäure:: 2,7 %
- Mizellgröße:: 30 mm
- pH-Wert:: 5,9

## Patentansprüche

1. Stabile, zur parenteralen Verabreichung geeignete wäßrige Solubilisate von Carotinoiden und Vitaminen oder Vitaminderivaten, in denen das Carotinoid und die nicht wasserlöslichen Vitamine mit Hilfe eines nichtionogenen Emulgators micellar gelöst sind, wobei die Micellen kleiner 100 nm sind, dadurch gekennzeichnet, daß die Solubilisate, in Mengen bezogen auf das Solubilisat, zumindest ein Carotinoid in einer Konzentration von 0,1 bis 10 Gew.-%, die nicht wasserlöslichen, lipophilen Vitamine oder Vitaminderivate in einer Konzentration von 0,1 bis 20 Gew.-% und die nichtionogenen Emulgatoren in einer Konzentration von 1 bis 40 Gew.-% enthalten, mit der Maßgabe, daß der Gehalt der lipophilen Vitamine mindestens so groß wie der des Carotinoids ist.

2. Stabile Solubilisate gemäß Anspruch 1, dadurch gekennzeichnet, daß der Gehalt des Carotinoids 0,4 bis 6 Gew.-%, der lipophilen Vitamine 0,4 bis 10 Gew.-% und der nichtionogenen Emulgatoren 5 bis 25 Gew.-% beträgt.

3. Stabile Solubilisate gemäß Anspruch 1, dadurch gekennzeichnet, daß sie Carotinoide, lipophile Vitamine, hydrophile Vitamine sowie gegebenenfalls Mineralstoffe enthalten.

4. Solubilisate gemäß Anspruch 1, dadurch gekennzeichnet, daß sie ein Carotinoid, Tocopherol oder einen Tocopherolester, Ascorbinsäure, sowie gegebenenfalls Selenverbindungen und N-Acetylcystein enthalten.

5. Solubilisate gemäß Anspruch 1, dadurch gekennzeichnet, daß sie als Carotinoide β-Carotin, Lycopin, Astaxanthin, Canthaxanthin, Citranaxanthin, Zeaxanthin, Apocarotinal und/oder Apocarotinsäureester, als lipophile Vitamine Tocopherol, Tocopherolacetat, Tocopherolsuccinat, Retinal, Retinol, Retinolester, Retinsäure, Cholecalciferol und/oder Ergocalciferol und als nichtionogene Solubilisatoren solche mit einem HLB-Wert von 10 bis 20 ausgewählt aus der Gruppe bestehend aus Polyoxyethylenglyceroltriricinoleat, Polyoxyethylen-12-hydroxystearat, Polyoxyethylensorbitanfettsäureester und Polyoxyethylen-polyoxypropylen-Blockpolymer enthalten.

6. Solubilisate gemäß Anspruch 1, dadurch gekennzeichnet, daß sie unmittelbar oder nach Verdünnung mit einem physiologisch verträglichen Vehikel in Form von Ampullen, Fertigspritzen, Infusionslösungen, Tropflösungen oder Säften vorliegen.

7. Verfahren zur Herstellung von Solubilisaten gemäß Anspruch 1, dadurch gekennzeichnet, daß man das Carotinoid mit den liphophilen Vitaminen oder Vitaminderivaten und dem nichtionogenen Emulgator kurzzeitig auf Temperaturen über 120°C bis zur Lösung erhitzt und sofort mit Wasser oder einer wäßrigen Lösung der hydrophilen Bestandteile turbulent vermischt und die Mischung abkühlt.

8. Verfahren zur Herstellung von Solubilisaten gemäß Anspruch 1, dadurch gekennzeichnet, daß man allein das Carotinoid mit dem nichtionogenen Emulgator kurzzeitig auf Temperaturen auf über 120°C bis zur Lösung erhitzt und sofort mit einem getrennt hergestellten Solubilisat der lipophilen Vitamine turbulent vermischt und die Mischung abkühlt.
